# EUROPEAN PATENT APPLICATION

(11) **EP 1 839 694 A1**
(43) Date of publication of application: **03.10.2007**
(21) Application number: 07251387.2
(22) Date of filing: 30.03.2007
(51) Int. Cl.: A61M 5/142, A61M 5/172

(54) **Drug delivery systems and methods**

(30) Priority: 31.03.2006 US 394812
(71) Applicant: Lifescan, Inc., Milpitas, CA 95035 (US)
(72) Inventor: Hohl, David, Milipas, CA 95035 (US); Shipway, Ian, Ardmore Pennsylvania 19003 (US); Getz, Steven, Malvern Pennsylvania 19355 (US); Kraft, Ulrich, 65719 Hofheim (DE); Ebner, Manfred, 61440 Oberursel (DE); Niess, Thorsten, 63674 Altenstadt (DE); Binz, Joachim, 63814 Mainaschaff (DE); Muller-Pathle, Stephan, 64291 Darmstadt (DE)
(74) Representative: Tunstall, Christopher Stephen

(57) **Abstract**

The present invention provides systems for delivering medication with drug delivery devices such as drug pumps or the like. Drug delivery systems in accordance with the present invention include a remote controller (200) that wirelessly communicates with a drug delivery device (300). The remote controller can control an infusion pump in such a way that provides the user with better control of the amount of medication dispensed.

## Description

### Technical Field

The present invention relates to systems and methods for delivering medication with drug delivery devices such as drug pumps or the like. More particularly, the present invention relates to drug delivery systems that include a remote controller that wirelessly communicates with a drug delivery device.

### Background

The use of infusion pumps for various types of drug therapy is becoming more common, where these infusion pumps are used to automatically administer liquid medicant to a patient. The liquid medicant is supplied from a source of medicant and pumped into the patient via a catheter or other injection device. For example, diabetics can utilize external infusion therapy for delivering insulin using devices worn on a belt, in a pocket, or the like, with the insulin being delivered via a catheter with a percutaneous needle or cannula placed in the subcutaneous tissue. In addition, medication pump therapy is becoming more important for the treatment and control of other medical conditions, such as pulmonary hypertension, HIV and cancer. The manner in which the liquid is infused is controlled by the infusion pump, which may have various modes of infusion, such as a continuous mode in which the liquid medicant is continuously infused at a constant rate, or a ramp mode in which the rate of infusion gradually increases, then remains constant, and then gradually decreases.

Typically, the monitoring of an infusion pump is performed by reviewing a visual display means incorporated in the infusion pump, and the control of the infusion pump is performed by activating an input device, such as a keypad, incorporated with the infusion pump. Consequently, the monitoring and/or control of an infusion pump are performed at the same location at which the infusion pump is disposed. One drawback of this type of drug therapy is the inability to conceal an external infusion pump and catheter tubing from view. Many users desire to hide the external pump under clothing so as keep their medical condition private. However, this can be inconvenient or impractical, especially for diseases such as diabetes, since a user must have ready access to the external pump for periodic monitoring or administering extra amounts of medication (i.e., boluses during the course of the day). If a user has concealed the external pump, the user often must partially undress or carefully maneuver the external pump to a location that permits access to the display and keypad.

In response to these issues, systems have been developed to allow a user to control the infusion pump through a remote controller that communicates with the pump via a wireless communications link, for example. In this way, a user can dispense medications without needing to physically access the pump, thereby making infusion pump therapy more convenient for the user. Many times, the user can start a process of dispensing a dosage of medication by simply providing a small number of commands to the pump via the remote controller, which will dispense the entire desired volume of medication without interruption. Situations may occur, however, in which it may be desirable for a user to stop or start the dispensing process in an urgent manner to ensure that the proper therapeutic amount of the medication enters the body. For example, a user that mistakenly initiates the dispensing of a bolus of medication may wish to abruptly stop the dispensing of medication, such as in a situation where a user mistakenly sends a bolus command with an inadvertently large bolus quantity. If the medication to be dispensed were insulin, the user would need to quickly cancel the bolus command to prevent a potentially hypoglycemic event from occurring.

In these types of urgent situations, the pump may be able to be stopped by pressing an appropriate navigation button on the pump or by removing the needle that dispenses the medication from the user's skin. One disadvantage to either of these approaches is that the navigation buttons and needle may be difficult to access due to the location of these items underneath a user's clothing, which can create a significant time delay. Removal of the needle is also inconvenient because the needle insertion process must then be repeated. In another alternative, the pump may be stopped by pressing a navigation button on the remote controller. However, under certain situations, wireless communication can be lost which would prevent a remote controller from being able to stop the pump. Thus, it is desirable to provide a medication dispensing system that allows for use of a remote controller to control an infusion pump in such a way that provides the user with better control of the amount of medication dispensed.

### Summary

In an aspect of the present invention, a method of delivering a bolus of insulin using a remote controller is provided. The method comprises the steps of: establishing a communication link between the remote controller and an infusion pump that contains insulin; providing a bolus value to the pump with the remote controller, the bolus value indicating a total amount of insulin to be dispensed; dispensing a predetermined portion of the total amount of insulin from the pump; and dispensing an additional predetermined portion of the total amount of insulin from the pump based on information comprising receipt of a continue command from the remote controller.

In another aspect of the present invention, a method of delivering a bolus of insulin using a remote controller and pump is provided where an alarm is triggered if a signal is not received by the pump within a predetermined period of time. The method comprises the steps of: establishing a communication link between the remote controller and an infusion pump that contains insulin; providing a bolus value to the pump with the remote controller, the bolus value indicating a total amount of insulin to be dispensed; dispensing a predetermined portion of the total amount of insulin from the pump; and triggering an alarm if a signal comprising a continue command is not received by the pump within a predetermined period of time.

In another aspect of the present invention, a method of delivering a bolus of insulin using a remote controller and pump is provided where dispensing of medication is terminated if a signal is not received by the pump within a predetermined period of time. The method comprises the steps of: establishing a communication link between the remote controller and an infusion pump that contains medication; providing a bolus value to the pump with the remote controller, the bolus value indicating a total amount of medication to be dispensed; dispensing a predetermined portion of the total amount of medication from the pump; and terminating dispensing of medication from the pump if a signal comprising a continue command is not received by the pump within a predetermined period of time.

In yet another aspect of the present invention, a method for dispensing a predetermined quantity of medication as a bolus is provided. The method comprises the steps of: inputting the predetermined quantity of medication into a remote controller; wirelessly transmitting information comprising the predetermined quantity of medication from the remote controller to a pump; dispensing a predetermined first portion of the quantity of medication from the pump; and dispensing an additional predetermined portion of the quantity of medication from the pump in response to receiving a wireless signal from the remote controller wherein the wireless signal comprises instructions to continue dispensing.

### Brief Description of the Drawings

These and other features, aspects, and advantages of the present invention will become better understood with regard to the following description, appended claims, and accompanying drawings where:
Figure 1 is a plan view of an exemplary remote controller in accordance with the present invention;
Figure 2 is a perspective view of the remote controller of Figure 1;
Figure 3 is a plan view of an exemplary test strip that can be used with the remote controller shown in Figures 1 and 2;
Figure 4 is a perspective view of a pump in accordance with the present invention;
Figure 5 is a schematic diagram showing certain functional aspects of the remote controller of Figure 1 and the pump of Figure 2 and showing in particular an aspect of wireless communication between the remote controller and pump;
Figure 6 is a schematic flow chart showing an exemplary method for dispensing a bolus of medication in accordance with the present invention;
Figure 7 is a schematic flow chart showing the method of Figure 6 and showing in particular steps for reestablishing a lost wireless signal in accordance with the present invention;
Figure 8 is a schematic diagram showing a sequence of commands and responses using a status request and continue command in accordance with the present invention; and
Figure 9 is a schematic diagram showing a sequence of commands and responses using only the continue command in accordance with the present invention.

### Detailed Description

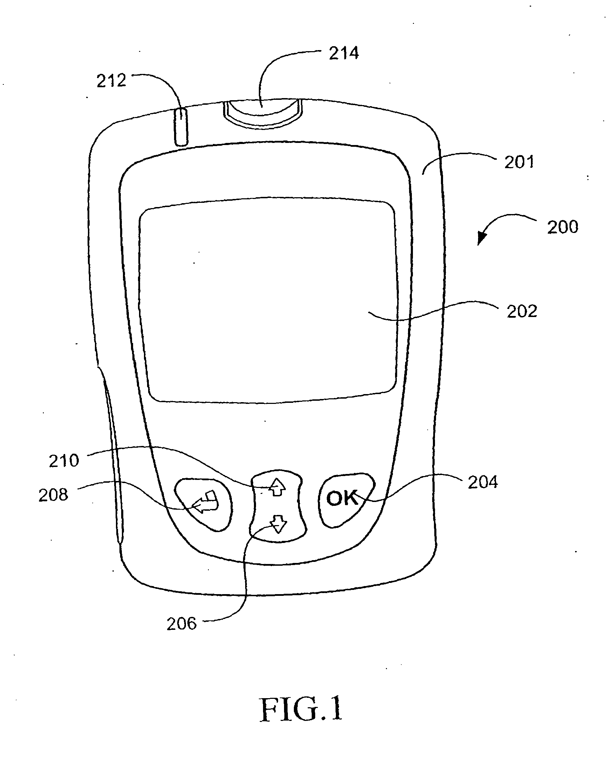

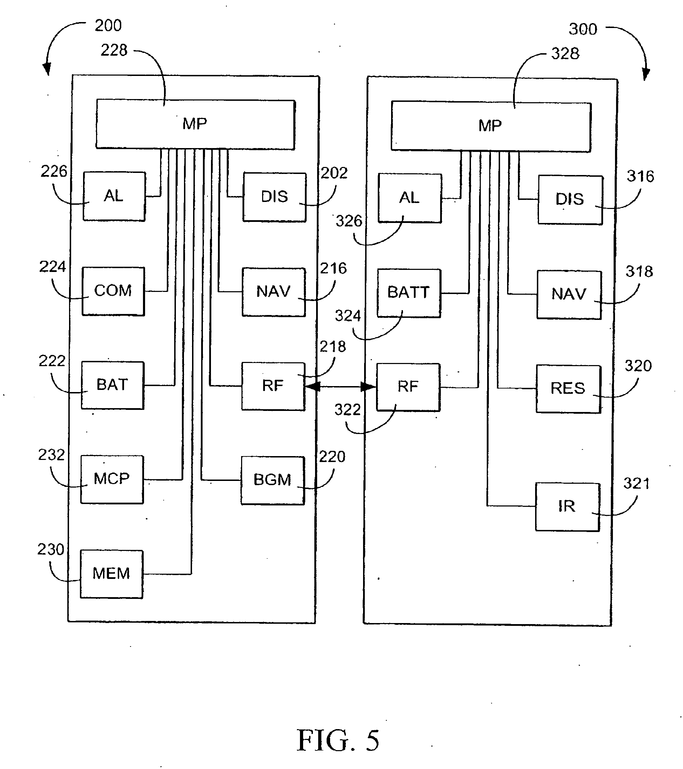

Figure 1 is a plan view of an exemplary remote controller 200 in accordance with the present invention. Remote controller 200, as shown, includes a first housing 201, a display 202, an OK button 204, a down button 206, back button 208, an up button 210, light emitting diode (LED) 212, and strip port connector (SPC) 214. Remote controller 200 is schematically shown in Figure 5 to further include functional components including navigational buttons (NAV) 216, a radio frequency module (RF) 218, a blood glucose measurement (BGM) module 220, a battery (BAT) 222, a wired communication port (COM), an alarm (AL) 226, a microprocessor (MP) 228, a memory portion (MEM) 230, and a memory chip port (MCP). Preferably, first housing 201 is ergonomically designed to be handheld and to incorporate the functional circuitry required for measuring glucose episodically and provide wireless communication with pump 300.

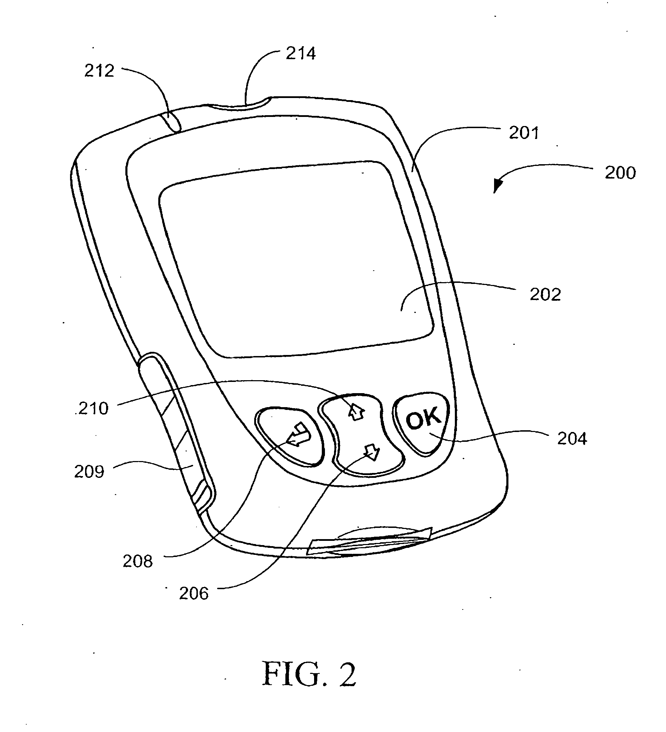

Figure 2 is a perspective view of remote controller 200 that further illustrates port cover 209. Preferably, port cover 209 comprises an elastomeric material that covers a wired connection port (not shown) and a memory chip port (not shown). Examples of a wired connection port include a universal serial bus (USB) or IEEE RS 232. Examples of memory suitable for insertion into a memory chip port include a flash memory such as a SIMM card, a SmartCard, Smart Media, and the like.

Display 202 preferably comprises a liquid crystal display (LCD) to show both textual and graphical information to a user. A user interface (UI) may comprise a software driven menu that can be shown on display 202 that enables the user to operate remote controller 200. A user can navigate through the UI using navigation buttons 216 which include up button 210, down button 206, OK button 204, and back button 208. Preferably, the UI allows a user to perform functions including operating pump 300, querying the status of pump 300, measuring glucose episodically, and displaying data on display 202 from remote controller 200 and/or pump 300 (e.g. glucose concentration versus time).

Microprocessor 228 preferably controls display 202, navigational buttons 216, RF module 218, blood glucose measurement module 220, wired communication port 224, first alarm 226, and memory chip port 232. Microprocessor 228 further preferably provides the capability to perform various algorithms for the management of a medical treatment. Examples of such algorithms include a predictive algorithm for a user's glucose concentrations (e.g. an algorithm that predicts a user's glucose concentration in the future) and a bolus calculator. A bolus is a predetermined amount of a medication that is dispensed over a relatively short time. In the case of a bolus calculator, microprocessor 228 preferably can process inputs such as food data (e.g. carbohydrates) that may be entered manually using first navigation buttons 216, or via wired communication port from a personal computer or like device. Additionally, blood glucose data can be provided to microprocessor 228 directly from the blood glucose measurement module 220. Using the inputted food data and glucose measurement data, a bolus of insulin can be determined, and shown on display 202, and the bolus amount can be transmitted wirelessly from remote controller 200 to pump 300. This enables pump 300 to dose an appropriate amount of insulin to a user while at the same time reducing the amount of user interactions with pump 300.

RF module 218 of remote controller 200 provides for bi-directional communication to pump 300 and potentially other devices such as a continuous glucose monitor, a personal computer, a personal digital assistant, a cell phone, insulin pen, or a second pump which may dispense glucose. Exemplary frequencies which may be suitable for use with RF module 218 are about 433 MHz, about 863 MHz, about 903 MHz, and about 2.47 GHz. RF module 218 may include a commercially available component such as a Chipcon CC 1000, an antenna, and a RF impedance matching network. RF module 218 may send commands to pump 300 such as a basal pumping rate, duration of pumping, and bolus amounts. In addition, RF module 218 may receive data from pump 300. Such data can include information indicating an occlusion or other error condition, an amount of insulin in reservoir, battery lifetime status, and historical insulin delivery information.

Wired communication port 224 provides the option of transferring data to or from an external device such as a personal computer. Wired communication port 224 may also be used to upgrade the software memory portion 230 of remote controller 200. Memory portion 230 preferably comprises a volatile memory type such as, for example, flash memory. Memory portion 230 preferably contains the application and system software for operating remote controller 200. Wired communication port 224 may then re-write memory portion 230 such that the entire application and system software is upgraded. This allows potential bugs in the software to be fixed and may be used to create added functionality in remote controller 200. In addition, a flash memory card may be inserted into memory chip port 232 for upgrading remote controller 200 without connecting it to a personal computer.

Remote controller 200 preferably includes alarm 226 which may be in a variety of forms to warn a user of various statuses that might need an actionable response. For example, alarm 226 may include an audio alarm (monophonic beeps or polyphonic tones), a vibratory alarm, or a LED 212 which may be a multi-colored LED that can illuminate red, yellow, and green lights. An alarm signal can be used to warn a user that there is a low glucose reading, a partially filled glucose test strip, a low reservoir of insulin, an occlusion in pump 300, a low battery status for pump 300, a low battery status for remote controller 200, an improperly filled test strip, or the like. For the previously mentioned situations in which a user may need to intervene because of a potentially dangerous situation, the alarm may be a vibration, audio signal, and/or LED 212 switching from green to red or from green to yellow.

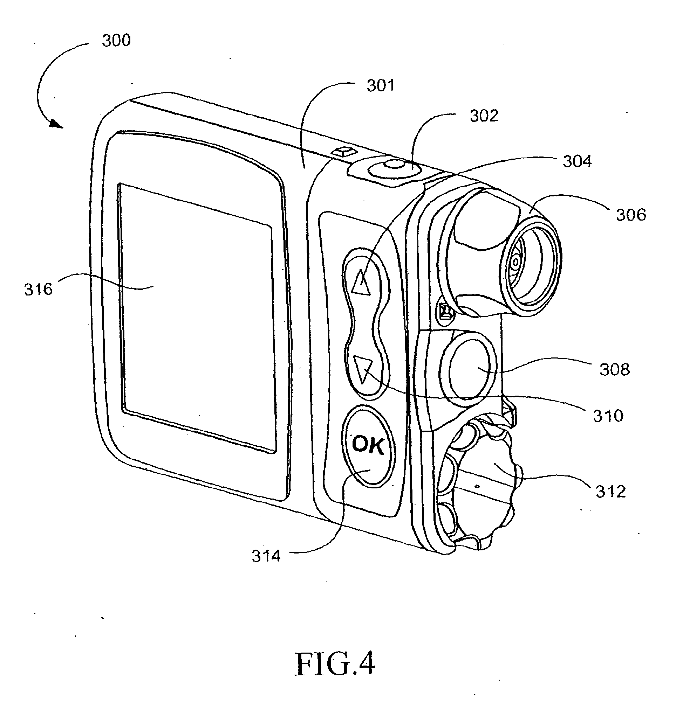

Figure 4 is a perspective view of a pump 300 in accordance with the present invention. Pump 300, as shown, includes a housing 301, a backlight button 302, an up button 304, a cartridge cap 306, a bolus button 308, a down button 310, a battery cap 312, an OK button 314, and a display 316. Pump 300 preferably comprises a pump suitable for use in dispensing medication such as insulin for improved diabetic therapies. For example, pump 300 may be similar to a commercially available pump from Animas, Corp. (Catalog No. IR 1200) except that pump 300 includes RF capabilities in accordance with the present invention. Housing 301 may comprise an RF transparent material and/or may be painted with an RF transparent paint. Pump 300 further preferably includes display (DIS) 316, navigational buttons (NAV) 318, a reservoir (RES) 320, an infrared communication port (IR) 321, a radio frequency module (RF) 322, a battery (BAT) 324, an alarm (AL) 326, and a microprocessor (MP) 328 as shown in Figure 5. Pump 300 and remote controller 200 preferably bi-directionally communicate using a wireless signal via RF module 218 and RF module 322.

Preferably, the antenna portion of RF module 218 is located within housing 201. Similarly, RF module 322 is preferably located within housing 301. In such a case, the material used for housing 201 and housing 301 is preferably RF transparent (i.e. does not absorb or interfere with RF signals). Further, if housing 201 or housing 301 is painted, the paint used is preferably RF transparent as well.

RF module 218 and RF module 322 further preferably include a communication protocol that enables remote controller 200 to communicate with a particular pump 300. Both remote controller 200 and pump 300 preferably have a unique identification code associated with their respective RF module 218 and RF module 322. This is desirable because under certain conditions, a second user with a second pump 300 may be in close proximity to the first user. It would be undesirable for the first user's remote controller 200 to cross communicate with the second user's pump 300. In order to avoid such cross communication, a remote controller 200 preferably initiates a pairing protocol before using pump 300 for the first time. When initiating the pairing protocol, remote controller 200 and pump 300 exchange their unique identification code (e.g. serial number). In all subsequent wireless communications, the correct unique identification code is preferably established before exchanging data.

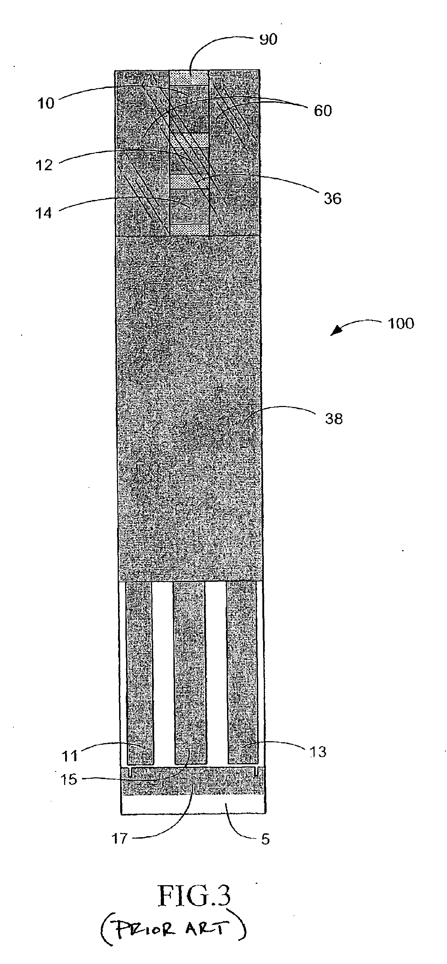

Remote controller 200 preferably comprises an integrated blood glucose meter that can measure glucose episodically using disposable test strips. An exemplary test strip 100 suitable for use in remote controller 200 is shown in Figure 3. Test strip 100 includes a conductive layer printed onto a substrate 5. The conductive layer includes a first contact 13, a second contact 15, a reference contact 11, and a strip detection bar 17 that may be used to electrically connect to strip port connector 214. The conductive layer further includes a first working electrode 12, a second working electrode 14, and a reference electrode 10 which are electrically connected, respectively, to first contact 13, second contact 15, and reference contact 11. Test strip further includes a clear hydrophilic film 36 which is bound by an adhesive 60 that forms a sample receiving chamber that allows blood to be dosed at inlet 90. An exemplary test strip is the commercially available OneTouch Ultra test strip from LifeScan, Inc. in Milpitas, California, U.S.A.

A reagent layer (not shown) is provided on first working electrode 12, second working electrode 14, and reference electrode 10. Reagent layer may include chemicals such as a redox enzyme and mediator which selectivity reacts with glucose. During this reaction, a proportional amount of a reduced mediator can be enzymatically generated which is measured electrochemically. This allows a current to be measured that is proportional to the glucose concentration. Examples of reagent formulations or inks suitable for use in making reagent layer 22 can be found in U.S. Patent Nos. 5,708,247 and 6,046,051 and Published International Applications WO01/67099 and WO01/73124, all of which are fully incorporated by reference herein for all purposes.

Preferably, in addition to measuring glucose episodically, remote controller 200 can also wirelessly communicate with pump 300. In use, remote controller 200 sends commands to pump 300 to dispense a fluid or medication for a pre-determined time period, rate, and/or volume. Preferably, a user selects from a menu of basal programs that have been programmed on pump 300. The user can also preferably set a basal rate, a bolus dose, and a combination thereof as commands to pump 300 from remote controller 200. Remote controller 200 receives data from pump 300 such as the status of the dispensing of medication (e.g. the dispense rate, amount of medication remaining in pump 300, or the proportion of medication delivered based on the amount programmed).

During routine use, a user may send a bolus command from remote controller 200 to pump 300 to initiate the dispensing of an insulin bolus. However, under certain circumstances, a user may mistakenly send a bolus command where the bolus was inadvertently too large. For this case, the user would need to cancel the bolus command to prevent a potential hypo-glycemic event from occurring. Pump 300 can be stopped by pressing the appropriate navigation button 318 on pump 300, removing the needle from the user's skin that dispenses insulin, or by pressing the appropriate navigation button 216 on remote controller 200. Stopping pump 300 by using navigation button 318 may be inconvenient because pump 300 may be inconspicuously worn underneath a user's clothing. Removing the needle which dispenses insulin may be inconvenient because the needle may be difficult to access and because the insertion process must then be repeated. Stopping pump 300 by using remote controller 200 obviates the problems associated with using navigation buttons 318 or removing the needle from a user's skin. However, stopping pump 300 could be problematic if there is a loss of wireless communication.

A typical bolus of insulin ranges from about 0.5 units to about 10 units and a typical bolus delivery period for a 10 unit bolus would be about 20 seconds. This provides a user with a relatively short time window of about 20 seconds or less to cancel an undesired bolus command. This further shows that pressing the appropriate navigation button 318 or removing the needle from the user's skin would be inconvenient because the user would have to act quickly. In such a situation, using remote controller 200 would be a more expedient way to cancel the bolus command.

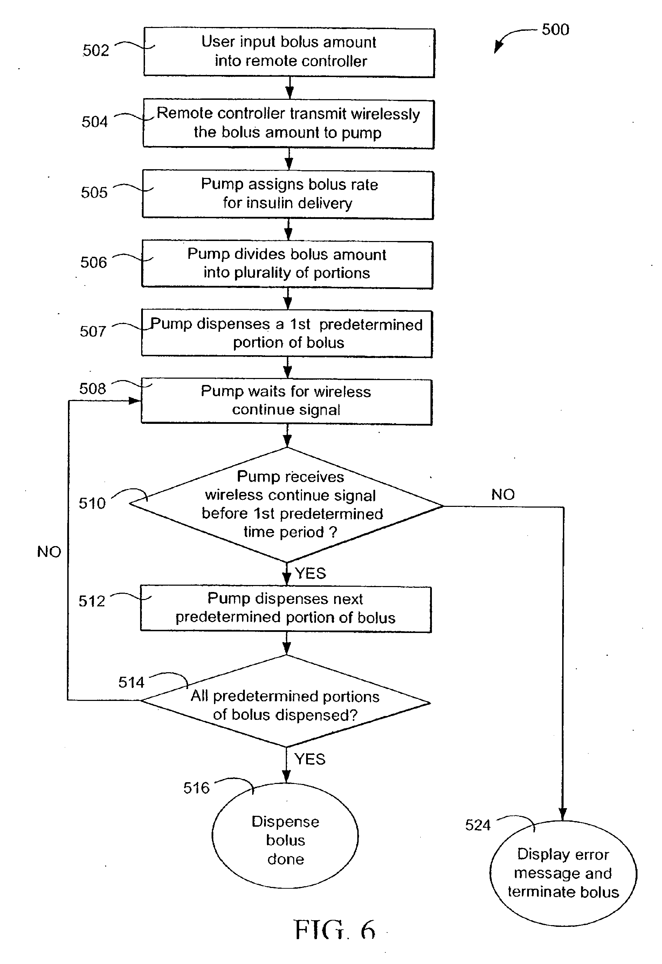

Figure 6 shows an exemplary method 500 for dispensing a bolus of medication such as insulin in accordance with the present invention. As shown, method 500 includes a step 502 in which a user inputs a predetermined quantity or a bolus amount into remote controller 200 using navigation buttons 216. Remote controller 200 then wirelessly transmits the bolus amount to pump 300 as shown in step 504. Depending on the magnitude of the bolus amount, pump 300 assigns a pre-determined bolus delivery rate for insulin delivery that typically delivers a bolus in less than about 20 seconds as shown in step 505. In addition, microprocessor 328 divides the bolus amount into a plurality of predetermined portions as shown in step 506. Each of the plurality of predetermined portions are preferably sized so they range from about 0.10 units to about 1.0 units. Preferably, the predetermined portions are equally sized. For example, a bolus amount of 3.0 units can be divided into 3 portions of 1.0 unit each, 6 portions of 0.50 units each, 12 portions of 0.25 units each, etc. The plurality of predetermined portions do not need to be equally sized and may be different. For example, a bolus amount of 3.0 units can be divided in to 2 portions of 1.0 unit each and 2 portions of 0.50 units each.

Pump 300 then dispenses a first predetermined portion of the bolus as shown in step 507. Next, pump 300 waits for a wireless signal from remote controller 200 to continue dispensing as shown in step 510. Once pump 300 receives wireless signal 400 to continue dispensing from remote controller 200, pump 300 then dispenses a subsequent predetermined portion as shown in step 512, which in this case is a second predetermined portion. If pump 300 does not receive a wireless signal to continue dispensing within a first predetermined waiting period, then display 202 preferably shows an error message to the user indicating that wireless communication has been lost and that the bolus has been terminated. The first predetermined waiting period preferably ranges from about 10 seconds to about one minute.

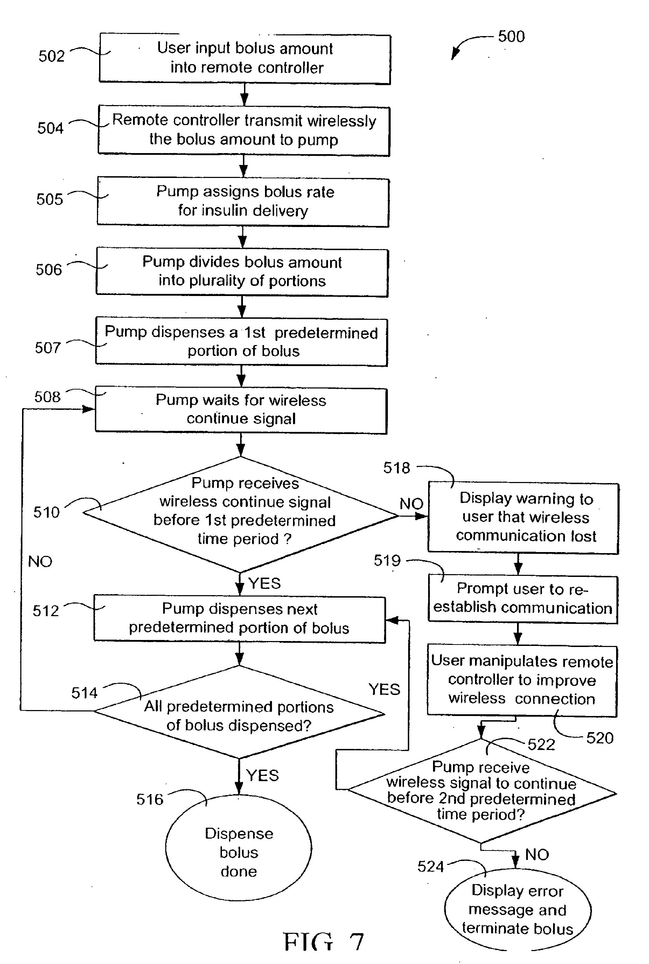

As shown in Figure 7, if pump 300 does not receive a wireless signal to continue dispensing within the first predetermined waiting period, the user is preferably prompted to try to reestablish wireless communication in step 519. In step 520, the user preferably manipulates the position of remote controller 200 in an attempt to improve wireless transmission or to generally check the meter for problems. For example, a user may physically move remote controller 200 closer to pump 300 or move remote controller 200 such that large metal objects do not interfere with the wireless transmission. In step 522, pump 300 waits for a wireless signal to continue dispensing within a second predetermined waiting period from remote controller 200. Preferably, the second predetermined waiting period is about one minute or less. If the user's intervention sufficiently improved the wireless signal transmission so that a continue command can be received, pump 300 then dispenses the next predetermined portion of the bolus as shown in step 512. If the wireless signal transmission is not sufficiently improved within the second predetermined waiting period, remote controller 200 preferably displays an error message and terminates the dispensing of the bolus as shown in step 524.

After pump 300 finishes dispensing a predetermined portion in step 512, pump 300 determines whether all portions have been dispensed as shown in step 514 as shown in Figure 6 and 7. If there are still remaining predetermined portions to be pumped, then the next step would be step 508 in which pump 300 waits for a wireless signal to continue. If all of the predetermined portions have been dispensed, then method 500 for dispensing a bolus is finished as shown in step 516. As illustrated, in method 500 a bolus is dispensed into the body only if wireless communication is maintained between remote controller 200 and pump 300 throughout the bolus process. Pump 300 must receive wireless commands to continue before dispensing a portion of the bolus. This method ensures that the bolus can be stopped quickly using remote controller 200. If this is not possible because of a loss of wireless communication, then method 500 will stop the bolus process.

Various modes can be used in accordance with the present invention for remote controller 200 to send continue commands. In a first exemplary mode that is described below in Example 1, remote controller 200 sends a recurring polling command interrogating pump 300 in regards to its status. Remote controller 200 may send the polling command in an asynchronous or synchronous manner. When pump 300 communicates to remote controller 200 that a predetermined portion of a bolus has been dispensed after receiving a polling command, remote controller 200 then sends the continue command. Remote controller 200 continually polls pump 300 to determine when another continue command needs to be sent to pump 300 so that all of the predetermined portions can be dispensed.

In a second exemplary mode which is described below in Example 2, remote controller 200 does not send any polling commands, but instead continually sends out continue commands at a predetermined frequency until the bolus is complete or until the bolus is terminated by remote controller 200. In this mode, if a continue command was sent to pump 300 before a predetermined portion has been dispensed, pump 300 ignores the continue command. Once pump 300 has finished dispensing a predetermined portion, it can then receive the continue command from remote controller 200. This second mode is more simplistic than the first mode because it does not use the polling command. However, the second mode sends several continue commands some of which are ignored by pump 300 if it has not finished dispensing a predetermined portion at that point in time.

### Example 1

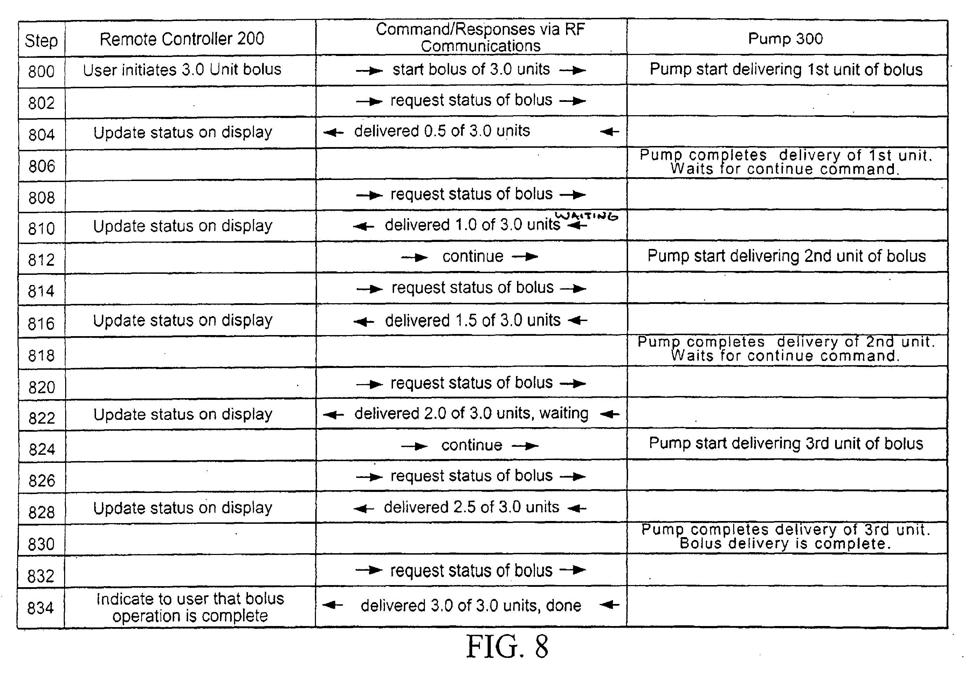

Figure 8 is a schematic showing a sequence of commands and responses using a status request (e.g. polling) and a continue command in accordance with the present invention. In step 800, a 3.0 unit bolus of insulin is selected or input using the user interface on remote controller 200. Remote controller 200 sends a command to the pump 300 via wireless signal 400, which instructs pump 300 to start a bolus of 3.0 units. Pump 300 divides the bolus into three predetermined portions of one unit each. Pump 300 then begins delivering the first predetermined portion of the bolus into the user's body.

In step 802, remote controller 200 sends a command requesting the status of the bolus. In this example, the polling step is performed on a recurring basis. At step 804, pump 300 has only delivered 0.5 units when the command is received causing pump 300 to send a response indicating that 0.5 of the requested 3.0 units has been delivered, and remote controller 200 updates the bolus delivery status information on first display 202. In step 806, pump 300 continues to deliver insulin until a total of 1.0 unit is delivered. At the end of step 806, pump 300 waits to receive a continue command from remote controller 200. In step 808, remote controller 200 sends another command requesting the status of the bolus. In step 810, pump 300 sends a response to the polling command indicating that 1.0 unit was delivered, and that it was now waiting for a continue command, and remote controller 200 updates the bolus delivery status information on first display 202. In step 812, remote controller 200 then sends a continue command to the pump 300, which then allows pump 300 to continue delivering the second predetermined portion of the bolus. In summary, steps 802 to 812 cause the first predetermined portion of the bolus to be dispensed, ensures that remote controller 200 and pump 300 can still wirelessly communicate, and then initiates the dispensing of the next predetermined portion of the bolus.

In Example 1, steps 814 to 834 enable all three predetermined portions to be delivered if wireless communication is not lost. In step 814, remote controller 200 sends a command requesting the status of the bolus. At step 816, pump 300 has delivered 1.5 units when the command is received causing pump 300 to send a response indicating that 1.5 of the requested 3.0 units has been delivered, and remote controller 200 updates the bolus delivery status information on first display 202. In step 818, pump 300 continues to deliver insulin until a total of 2.0 units have been delivered. At the end of step 818, pump 300 waits to receive a continue command from remote controller 200. In step 820, remote controller 200 sends another command requesting the status of the bolus. In step 822, pump 300 sends a response indicating that 2.0 units has been delivered, and that it is now waiting for a continue command, and remote controller 200 updates the bolus delivery status information on first display 202. In step 824, remote controller 200 then sends a continue command to the pump 300, which then allows pump 300 to continue delivering the third predetermined portion of the bolus.

In step 826, remote controller 200 sends a command requesting the status of the bolus. At step 828, pump 300 has delivered 2.5 units when the command was received causing pump 300 to send a response indicating that 2.5 of the requested 3.0 units has been delivered, and remote controller 200 updates the bolus delivery status information on first display 202. In step 830, pump 300 continues to deliver insulin until 3.0 units has been delivered. In step 832, remote controller 200 sends another command requesting the status of the bolus. In step 834, pump 300 sends a response indicating that 3.0 units has been delivered, and that it is now done delivering all pre-determined portions of the bolus.

### Example 2

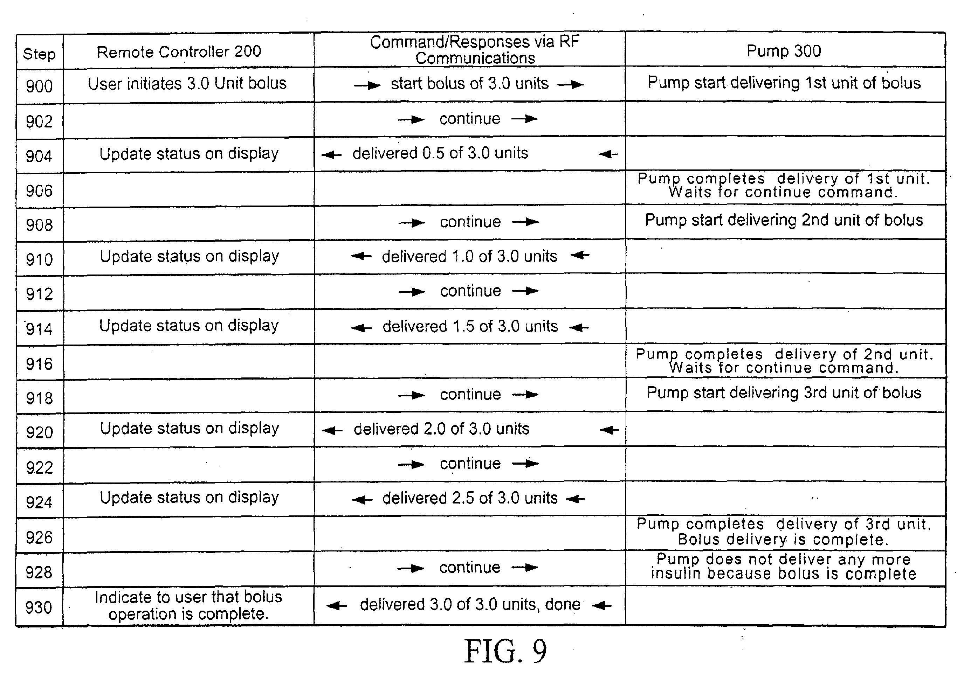

Figure 9 is a schematic showing an exemplary sequence of commands and responses using only the continue command in accordance with the present invention. In this example, there is no polling command from remote controller 200 to pump 300 as in Example 1. In step 900, a 3.0 unit bolus of insulin is selected using the user interface on remote controller 200. Remote controller 200 sends a command to the pump 300 via wireless signal 400, instructing pump 300 to start a bolus of 3.0 units. Pump 300 divides the bolus into three predetermined portions of one unit each. Pump 300 then begins delivering the first predetermined portion of the bolus into the user's body.

In step 902, remote controller 200 sends a continue command. In this example, the continue command may be performed either on a recurring basis or asynchronously. If a recurring signal is sent, it preferably has a frequency in the range of about 0.5 second to 1 second. For step 902, the continue command is received by pump 300 which causes it to respond with an indication of how much of the bolus has been delivered. At step 904, pump 300 sends a response indicating that 0.5 unit of the requested 3.0 units had been delivered, and remote controller 200 updates the bolus delivery status information on first display 202. In step 906, pump 300 continues to deliver insulin until a total of 1.0 unit has been delivered. At the end of step 906, pump 300 waits to receive a continue command from remote controller 200. In step 908, remote controller 200 sends another continue command. Once pump 300 received the continue command in step 908, it starts delivering the second predetermined portion of the bolus into the user's body. Next, pump 300 responds with an indication of how much of the bolus has been delivered in step 910 which in this case is 1.0 unit of the requested 3.0 units, and remote controller 200 updates the bolus delivery status information on first display 202. For step 912, the continue command is sent by remote controller 200 and received by pump 300. In step 914, pump 300 responds with an indication of how much of the bolus has been delivered which in this case was 1.5 units of the requested 3.0 units, and remote controller 200 updates the bolus delivery status information on first display 202. In step 916, pump 300 continues to deliver insulin until 2.0 units has been delivered and then waits for a continue command. For step 918, the continue command is sent by remote controller 200 and received by pump 300. Next, pump 300 responds with an indication of how much of the bolus had been delivered in step 920 which in this case was 2.0 units of the requested 3.0 units, and remote controller 200 updates the bolus delivery status information on first display 202. For step 922, the continue command is sent by remote controller 200 and received by pump 300. Next, pump 300 responds with an indication of how much of the bolus has been delivered in step 924 which in this case is 2.5 units of the requested 3.0 units, and remote controller 200 updates the bolus delivery status information on first display 202. In step 926, pump 300 continues to deliver insulin until a total of 3.0 units have been delivered which is the end of the bolus. For step 928, the continue command is sent by remote controller 200 and received by pump 300. However, pump does not dispense any more insulin because the bolus has been completely delivered. Pump 300 responds with an indication that 3.0 units of the requested 3.0 units was dispensed indicating that the bolus was completely delivered, and remote controller 200 updates first display 202 with an indication that the bolus is complete.

The present invention has now been described with reference to several embodiments thereof. The entire disclosure of any patent or patent application identified herein is hereby incorporated by reference. The foregoing detailed description and examples have been given for clarity of understanding only. No unnecessary limitations are to be understood therefrom. It will be apparent to those skilled in the art that many changes can be made in the embodiments described without departing from the scope of the invention. Thus, the scope of the present invention should not be limited to the structures described herein, but only by the structures described by the language of the claims and the equivalents of those structures.

## Claims

1. A system for delivering a bolus of insulin, the system comprising:
an infusion pump that contains insulin;
a remote controller;
means for establishing a communication link between the remote controller and the infusion pump;
means for providing a bolus value to the pump from the remote controller, the bolus value indicating a total amount of insulin to be dispensed;
means for dispensing a predetermined portion of the total amount of insulin from the pump; and
means for dispensing an additional predetermined portion of the total amount of insulin from the pump based on information comprising receipt of a continue command from the remote controller.

2. The system of claim 1, further comprising means for repeatedly dispensing an additional predetermined portion of the total amount of insulin from the pump until the total amount of insulin is dispensed.

3. The system of claim 1, wherein the communication link comprises a wireless signal transmitted between the remote controller and the infusion pump.

4. The system of claim 1, wherein the remote controller and infusion pump communicate via a unique identification code.

5. The system of claim 1, further comprising means for providing a bolus value to the remote controller.

6. The system of claim 1, wherein the remote controller comprises a blood glucose meter.

7. The system of claim 1, further comprising means for continuously transmitting a signal comprising a continue command wherein the signal is transmitted from the remote controller to the pump.

8. A system for delivering a bolus of insulin, the system comprising:
an infusion pump that contains insulin;
a remote controller;
means for establishing a communication link between the remote controller and the infusion pump;
means for providing a bolus value to the pump with the remote controller, the bolus value indicating a total amount of insulin to be dispensed;
means for dispensing a predetermined portion of the total amount of insulin from the pump; and
means for triggering an alarm if a signal comprising a continue command is not received by the pump within a predetermined period of time.

9. The system of claim 8, wherein the predetermined period of time is between 1 second and 60 seconds.

10. The system of claim 8, further comprising means for terminating dispensing of insulin from the pump based on the alarm.

11. The system of claim 10, further comprising means for waiting for a second predetermined period of time before terminating dispensing of insulin from the pump.

12. A system for delivering a bolus of medication, the system comprising:
an infusion pump that contains insulin;
a remote controller;
means for establishing a communication link between the remote controller and the infusion pump;
means for providing a bolus value to the pump with the remote controller, the bolus value indicating a total amount of medication to be dispensed;
means for dispensing a predetennined portion of the total amount of medication from the pump; and
means for terminating dispensing of medication from the pump if a signal comprising a continue command is not received by the pump within a predetermined period of time.

13. The system of claim 12, wherein the medication comprises insulin.

14. The system of claim 12, wherein the communication link comprises a wireless signal transmitted between the remote controller and the infusion pump.

15. The system of claim 12, further comprising means for triggering an alarm in response to terminating dispensing of medication.

16. A system for dispensing a predetermined quantity of medication as a bolus, the system comprising:
a pump that contains medication;
a remote controller;
means for inputting the predetermined quantity of medication into the remote controller;
means for wirelessly transmitting information comprising the predetermined quantity of medication from the remote controller to the pump;
means for dispensing a predetermined first portion of the quantity of medication from the pump; and
means for dispensing an additional predetermined portion of the quantity of medication from the pump in response to receiving a wireless signal from the remote controller wherein the wireless signal comprises instructions to continue dispensing.

17. The system of claim 16, further comprising means for dispensing the entire quantity of medication.

18. The system of claim 16, further comprising means for continuously providing a signal comprising the instructions to continue dispensing wherein the signal is transmitted from the remote controller to the pump.

19. The system of claim 16, further comprising means for providing information comprising status of the pump to the remote controller.

20. The system of claim 16, wherein the medication comprises insulin.
